# EUROPEAN PATENT SPECIFICATION

(11) **EP 1 931 223 B1**
(45) Date of publication and mention of the grant of the patent: **04.03.2009**
(21) Application number: 05808556.4
(22) Date of filing: 07.10.2005
(51) Int. Cl.: A23L 1/30, A23L 2/52, A61P 21/06

(54) **NUTRITION COMPRISING BETAINE AGAINST MUSCLE WASTING**
BETAINHALTIGE NAHRUNG GEGEN MUSKELSCHWUND
ALIMENT QUI COMPREND UNE BÉTAÏNE CONTRE UNE PERTE MUSCULAIRE

(43) Date of publication of application: 18.06.2008
(73) Proprietor: N.V. Nutricia, 2712 HM Zoetermeer (NL)
(72) Inventor: PETERS, Johannes, Adrianus, Cornelis, NL-2586 BX Den Haag (NL); VAN NORREN, Klaske, NL-6871 LP Renkum (NL); GORSELINK, Marchel, NL-7335 GR Apeldoorn (NL); HAGEMAN, Robert, Johan, Joseph, NL-6705 CT Wageningen (NL)
(74) Representative: Swinkels, Bart Willem
(86) International application number: PCT/NL2005/050010
(87) International publication number: WO 2007/043857

(56) References cited:
- US-A1- 2003 091 615
- US-A1- 2004 191 388
- KISHI T ET AL: "Effect of betaine on S-adenosylmethionine levels in the cerebrospinal fluid in a patient with methylenetetrahydrofolate reductase deficiency and peripheral neuropathy." JOURNAL OF INHERITED METABOLIC DISEASE. 1994, vol. 17, no. 5, 1994, pages 560-565, XP008065071 ISSN: 0141-8955
- DATABASE MEDLINE [Online] US NATIONAL LIBRARY OF MEDICINE (NLM), BETHESDA, MD, US; July 1989 (1989-07), YAMANAKA N ET AL: "[A case of motor and sensory neuropathy with elevated serum lactate and pyruvate which responded to large dose of coenzyme Q10 therapy]" XP002384530 Database accession no. NLM2805512 & RINSH SHINKEIGAKU = CLINICAL NEUROLOGY. JUL 1989, vol. 29, no. 7, July 1989 (1989-07), pages 885-889, ISSN: 0009-918X

## Description

### FIELD OF THE INVENTION

The invention relates to a nutritional composition for the treatment of muscle wasting in patients in need thereof. In particular the invention relates to a complete nutritional composition for patients with muscle wasting. Suitably the nutrition is in the form of a drink, powder or bar.

### BACKGROUND OF THE INVENTION

In patients with muscle wasting, the balance normally present between protein breakdown and protein synthesis is disturbed. This leads to involuntary weight loss of at least 5% of total body weight. Muscle wasting can occur in the terminal phase of cancer, depending on the type of cancer, but also after experiencing severe trauma or surgery and in association with ageing, e.g. persons suffering from dementia, severe illnesses like chronic inflammation, as in patients with chronic obstructive pulmonary disease (COPD) and severe infectious diseases like AIDS, and burns. Depletion of lean body muscle mass has been observed in people at all stages of HIV infection. Muscle wasting can be distinguished from starvation in that the former results in body-fat content preservation while body cell mass is depleted. This progressive wasting may result solely from the underlying HIV infection. Unintended and progressive weight loss of at least 5% of body weight eventually in combination with weakness, or a significant loss of body cell mass are indicators for the existence of muscle wasting. A lower body weight is often associated with an increased mortality, but also with more frequent complications such as infections, and decreased appetite, increased muscle weakness and fatigue.

WO9402036 discloses a composition that provides for sustained energy and nutrition to support an anabolic physiological state in humans. It is stated that this is beneficial for patients with physiological stress that often accompanies amongst others, cachexia. This document is therefore dealing with the problem to provide a sustained energy and anabolic nutritional composition for patients with cachexia. The composition comprises a blend of simple sugars and more complex carbohydrates, partially hydrolyzed protein, and, at least, magnesium in the form of an amino acid chelate.

US2004043013 discloses compositions for the support of an uncoupling therapy. This therapy leads to decreased ATP production that is contrary to the effect the inventors of the current application want to induce by supplementing a nutrition comprising betaine and CoQ10. Example 3 discloses the use of a composition comprising betaine and CoQ10 for use by obese patients in order to induce weight reduction. This is the opposite of the claimed effect of the current invention. Example 12 of this document describes a composition for use in cancer patients. The proposed formulation in this example neither contain betaine nor CoQ10.

It is an object of the invention to provide a composition that contains an optimal selection of ingredients to improve the balance between protein breakdown and synthesis by improving the energy content of the cell.

### SUMMARY OF THE INVENTION

Patients with muscle wasting suffer from symptoms such as weight loss, decreased appetite, muscle weakness and fatigue, etc. The inventors of the present invention surprisingly found that patients with muscle wasting benefit substantially from consuming betaine or equivalents thereof, and preferably in combination with coenzyme Q10 or equivalents thereof. The benefits include decreased loss of total body mass, decreased loss of lean body mass, improvement of, hence less, fatigue and improved stamina. Use of the present invention is particularly beneficial for the treatment or reduction of muscle wasting that is associated with trauma, transplantations, severe ischaemia/reperfusion damage, bums and surgery and other conditions wherein large amounts of tissue need to be replaced. Also non-surgical patients with COPD, severe infectious diseases like HIV/AIDS and ageing patients, e.g. patients with dementia, will benefit from the present invention.

The inventors surprisingly found that the use of betaine (trimethyl glycine) significantly improves the glycogen energy storage in muscles. Coenzyme Q10 enhances the efficient use of the increased energy storage thereby supporting the effect of betaine and remitting the wasting of muscle tissue.

Improved intracellular energy storage in muscle tissues is beneficial for patients with muscle wasting and particularly beneficial for cancer patients with muscle wasting because this improves the symptoms associated with low intracellular energy levels in patients with muscle wasting mentioned above. The invention thus concerns the use of betaine equivalents for the manufacture of a nutritional composition for the treatment or reduction of the incidence of muscle wasting in patients in need thereof Preferably betaine is used in combination with coenzyme Q10 equivalents (CoQ10) for the manufacture of a nutritional composition for the treatment or reduction of the incidence of muscle wasting in patients in need thereof In one embodiment the invention concerns the treatment of cancer patients with muscle wasting. However, the use of the invention is beneficial for all patients that suffer from muscle wasting due to other causes such as trauma or surgery patients and patients suffering from COPD or HIV infection.

In a preferred embodiment CoQ10 and betaine are used in combination with a complete nutritional formula supplying sufficient protein, carbohydrates and fat to the patients to prevent the loss of or restore the loss of the patient's body mass.

### DETAILED DESCRIPTION OF THE INVENTION

As previously stated, muscle wasting disturbs the homeostasis that is normally present between protein breakdown and protein synthesis. This leads to involuntary weight loss of at least 5% of total body weight. Nutritional therapy according to the invention is indicated when significant weight loss (more than 5% in three months), or a significant loss of body cell mass (more than 5% in 3 months) has occurred. In addition, nutritional therapy should be considered when the Body Mass Index (BMI) is below 18.5 kg/m²).

The present invention provides nutritional compositions that can improve the balance between protein breakdown and protein synthesis and thereby lead to less muscle wasting symptoms, e.g. improvement in lean body mass.

Sufficient energy stores in muscle is therefore beneficial for improving the balance between protein breakdown and protein synthesis leading to improved lean body mass.

*Betaine* is also known as trimethylglycine, N-trimethylglycine, glycine betaine, glycocoll betaine, oxyneurine and lycine. Its chemical name is 1-carboxy-N,N,N-trimethylmethanaminium inner salt. The molecular formula of betaine is C₅H₁₁NO₂, its chemical formula is (CH₃)₃N⁺-CH₂COO⁻. The hydrochloride of betaine is known as betaine hydrochloride, betaine HCl and pluchine. Its chemical name is 1-carboxy-N,N,N-trimethylmethanaminium chloride.

Betaine-homocysteine methyltransferase (BHMT) is a zinc metalloenzyme that catalyzes the transfer of a methyl group from betaine to homocysteine in the formation of methionine. BHMT is found in the liver and kidneys and may also exist in brain tissue. Betaine can also lower homocysteine levels in some persons with primary hyperhomocysteinemia/homocystinuria via this enzyme. Betaine has also been found to lower homocysteine levels in some animal studies, again, via BHMT.

Betaine equivalents are defined as betaine, dimethylglycine, betaine inner salts in anhydrous and hydrated forms as well as the forms wherein the molecule is associated with inorganic acids like hydrochloric acid or organic acids like citrate, malate aspartate or sarcosine. In a preferred embodiment betaine, dimethylglycine or a combination thereof is used. In a most preferred embodiment betaine is used.

For the purpose of this invention a suitable natural source of betaine are plants belonging to the *Chenopodiaceae* family, E.g. molasses of sugar beets are naturally rich in betaine. The preferred dose of betaine is between 50 and 50000 mg per day more preferably between 100 and 10000 mg per day and most preferred between 1000 and 10000 mg per day. The preferred dose of other betaine equivalents corresponds to that of betaine calculated on a molar basis.

*Coenzyme Q10* (also known as CoQ10, Q10, vitamin Q10, ubiquinone, and ubidecarenone) is a benzoquinone compound synthesized naturally by the human body. The "Q" and the "10" in the name refer to the quinone chemical group and the 10 isoprenyl chemical subunits, respectively, that are part of this compound's structure. The term "coenzyme" denotes it concerns an organic (contains carbon atoms), nonprotein molecule necessary for the proper functioning of its protein partner (an enzyme or an enzyme complex). Coenzyme Q10 is used by cells of the body in a process known variously as aerobic respiration, aerobic metabolism, oxidative metabolism, or cell respiration. Through this process, energy for cell growth and maintenance is created inside cells in compartments called mitochondria. In human, supplementation doses and administration schedules can vary, but preferably are in the range of 10 to 2000 mg/day and more preferably 20 to 1000 mg/day and most preferably between 90 to 390 mg/day. Although the number of isoprenyl units is preferably 10, a similar activity in humans could be achieved by a number of 8, 9 or 12 units.

Collectively these compounds are referred to as CoQ10 equivalents. This term also includes synthetic equivalents as will be discussed below.

It is noted that CoQ10 can attain high plasma levels but may enter tissue rather poorly (Valliant F. et al, 1996. J. Bioenerg. Biomembr, 28:531-540). Therefore the present invention includes the use of synthetic equivalents of CoQ10, in particular of equivalents that show improved pharmacokinetics compared to CoQ10. One such an equivalent is idebenone (2,3-dimethoxy-5-methyl-6-(10-hydroxy)decyl-1,4-benzoquinone), which is a short-chain quinone that crosses cell membranes readily. Thus in one embodiment the present invention concerns the use of idebenone as CoQ10 equivalent.

Natural sources of CoQ10 equivalents that are suitable for this invention include extracts or isolates of fungi, yeasts or bacteria, canola oil or soybean oil.

A preferred embodiment includes the use of vitamin B6. This will improve the effectiveness of the nutritional formula when relatively low doses of coenzyme Q10 are used because this vitamin is essential for the biosynthesis of natural CoQ10 in the patient. When the dose is below 20 mg CoQ 10 per day, administration of more than 2 mg vitamin B6 increases endogenous CoQ 10 levels. Vitamin B6 can be administered in forms and methods that are known in the art, eg. as pyridoxine, pyridoxamine, pyridoxal or pyridoxal phosphate.

Without wanting to be bound by theory, the effect of betaine on glycogen formation may perhaps be attributed to the function of betaine as a methyl donor. The improved effect by applying the present invention can be quantified by measuring the rate of change in lean body mass or by measuring the glycogen energy stores. The improved effect may also be established by measuring the rate of use of betaine, e.g. by measuring ratios of metabolites of betaine, such as the ratio of dimethylglycine to sarcosine or glycine.

It was found that inclusion of folate in the product further increases the positive effects of betaine. In particular inclusion of more than 400 µg folate per daily dose appeared to be beneficial. Folate is also known as Vitamin B-9, folic acid; Pteroylglutamic acid. This component is able to increase the levels of tetrahydrofolate or methylated forms thereof in vivo. Folate can be used as folic acid in oxidized or reduced forms, or in mono- or polyglutamate forms, as well as folinic acid or methylated forms, together these are referred to as folate and folate equivalents. Thus in a further embodiment the invention includes the use of folate or equivalent thereof.

### Nutritional compositions

The nutritional compositions of the current invention can be beneficially used by patients with muscle wasting. The nutritional compositions can have a varying energy density, but because patients with muscle wasting often have an insufficient calorie intake, the energy density is preferably relatively high. When in the form of a liquid, the composition should comprise at least 0.9 kcal/ml and preferably at least 1.0 kcal/ml and most preferably at least 1.5 kcal/ml. At least 100 ml of the liquid composition is administered per day in a single dose, but also multiple doses, or enteral tube feeding regimens are possible. Particularly at home, nocturnal tube feeding is a very comfortable way of optimising the nutritional intake when the patient is suffering from a lack of appetite. A complete nutritional formula is especially beneficial because patients with muscle wasting often lack sufficient nutritional support and need macronutrients such as proteins, fat and carbohydrates as well as micronutrients such as vitamins and minerals.

In a preferred embodiment the nutritional composition comprises, betaine, CoQ10, at least 15 en% lipids, preferably, 15-70 en% lipids, 10-70 en% digestible carbohydrates and 10-35 en% protein, wherein the protein comprises all essential amino acids and the source of protein is chosen from the group of; dairy proteins, egg proteins, plant proteins and hydrolysates thereof. Preferably the concentration of betaine and/or equivalent thereof is between 0.01 and 500 mg/ml and the concentration CoQ10 and/or equivalent thereof is between 0.002 and 20 mg/ml. In an even more preferred embodiment, the complete formula has a lipid content of more than 15 en%, preferably between 27-80 en%, more preferably between 37-70 en% and most preferred between 42 and 70 en, 20-50 en% of digestible carbohydrates and 10-35 en% protein.

In a preferred embodiment the nutritional composition comprises a lipid fraction that provides EPA whereby the EPA content is more than 2 wt% of the lipid fraction.

Another preferred embodiment further comprises folate and/or vitamin B6, wherein the preferred concentration of folate is between 0.0005 and 0.05 mg/ml and wherein the concentration of vitamin B6 is between 0.001 and 0.07 mg/ml.

A protein composition that is rich in leucine is preferred. The amount of leucine is between 10.7 and 20 percent, more preferably between 11.2 and 19.1 weight percent of protein fraction. In order to limit the amount of free amino acids, preferably less than 20 wt% of the protein fraction is in the form of free amino acids. Preferably the ratio of leucine to the sum of valine + isoleucine + leucine is higher than 0.48.

It is especially preferred to use the composition in combination with a specific drug regimen. In particular muscle wasting is much decreased when a composition of this invention is used with doxorubicin, beta-blockers and statins.

### EXAMPLES

### Example 1

The inventors surprisingly found that the use of betaine (trimethylglycine) significantly improves the glycogen energy stores in muscles of normal healthy rats.

Table 1 shows that the resting glycogen levels in rat muscle tissue of rats supplemented with oral vanilla custard (control group) and with vanilla custard containing betaine. Betaine significantly increases the glycogen content of the muscle cells.

**Table 1. Resting glycogen levels in rats (µmol glycosyl units/g tissue)**

| Muscle type | No betaine supplement | With betaine supplement |
|---|---|---|
| EDL | 108±7 | 120+4^{*} |
| Soleus | 107±12 | 137±16^{*} |

| | | |
|---|---|---|
| * P<0.05 | | |

### Research design and methods

Experiments were performed on 8 male Wistar rats, 250-300 gram, which were housed individually, and had free access to water and food. All experimental procedures were approved by the Institutional Animal Care and Use Committee of the Netherlands, and complied with the principles of laboratory animal care. To determine the effect of oral betaine supplementation, the betaine group (n=4) received 1 ml vanilla custard each day in combination with 60 mg betaine (Sigma Aldrich), and the control group (n=4) received 1 ml vanilla custard. After 6 weeks of supplementation, the fast extensor digitorum longus, *(EDL) and slow-* (soleus) muscles were removed for further biochemical analysis and frozen in liquid nitrogen. Muscles were stored at -80 °C until analysis. Thereafter, muscles were freeze-dried during 3 days at -4 °C. The freeze-dried muscles were crushed to powder, 400 µl 1M HCl was added to approximately 10 mg tissue and hydrolysed at 98°C during 2 hours. After cooling down, 400 µl 1M NaOH was added to neutralize the samples and the samples were centrifuged 5 minutes at 13,000 rpm. Glycogen content was determined fluorimetrically by a glucose kit (Roche Diagnostics) and expressed as µmol glycosyl units·g⁻¹.

### Example 2 Nutritional supplement

Nutritional supplement for the treatment or prevention of muscle wasting can be in the form of a tablet comprising: 1 g betaine, 100 mg CoQ10 and 0.4 mg vitamin B6, 0.20 mg folic acid. An advisable daily dose is 2-4 tablets per day. One or more of the ingredients can be replaced by the equivalents thereof.

### Example 3 Clinical nutrition

One preferred embodiment of a liquid clinical nutrition for the treatment and prevention of muscle wasting comprises the following ingredients:

| Ingredients | energy percent | g/100ml |
|---|---|---|
| Protein | 26 | 10 |
| Carbohydrates | 47 | 18 |
| Lipids | 28 | 5.3 |
| Fibers | | 2.1 |
| Betaine | | 2 |
| CoQ10 | | 0.12 |
| Vit B6 | | 0.00068 |
| Folic acid | | 0.0004 |
| Other vitamins | | 0.1 to 1 times RDA levels |

Wherein the protein comprises all essential amino acids and the source of protein is chosen from the group of; dairy proteins, egg proteins, plant proteins and hydrolysates thereof and the lipids preferably comprise n-3 fatty acids such as EPA, preferably at least 5 wt% of total lipids and more preferably at least 10 wt% of the lipids comprise EPA.

### Example 4. Liquid formula for surgery patients with unintended loss of lean body mass comprising per 100ml.

| | |
|---|---|
| Casein | 6.1 g |
| L-Leucine | 0.2 g |
| Lipids | 4.9 g of which 0.2 g squalane and 0.1 g EPA |
| Betaine | 0.12 g |
| dimethylglycine | 0.02 g |

| vit minerals per 100 kcal: | |
|---|---|
| Vit. A | 82 µg |
| Carotenoids | 0.20 mg |
| Vit. D | 0.7 µg |
| Vit. E | 1.3 mg |
| Vit. K | 5.3 µg |
| Thiamine | 0.15 mg |
| Riboflavine | 0.16 mg |
| Niacine | 1.8 mg |
| Pantothenic acid | 0.53 µg |
| Vit.B6 | 0.17 µg |
| folic acid | 27 µg |
| Vit. B12 | 0.21 µg |
| Biotine | 4.0 µg |
| Vit. C. | 10 mg |

### Example 5. Nutritional bar for patients with unintended depletion of lean body mass caused by HIV infection comprising.

| **Raw Material** | **protein** | **carbs** | **fat** | **g/100g** |
|---|---|---|---|---|
| Colostrum | 15.00 | 2.10 | 0.80 | 21.04 |
| borage oil | 0.00 | 0.00 | 4.00 | 4.21 |
| EPA-DHA oil (Maruha) | 0.00 | 0.00 | 6.00 | 6.31 |
| Egg protein | 16.87 | 0.00 | 0.00 | 22.19 |
| Fructose syrup | 0.00 | 11.92 | 0.00 | 16.20 |
| glycerine | 0.00 | 3.83 | 0.00 | 4.05 |
| CoQ10 | | | | 0.35 |
| Vit B6 | | | | 0.0024 |
| Folic acid | | | | 0.0012 |
| Betaine | | | | 3.0 |

## Claims

1. Use of betaine, dimethylglycine or betaine inner salts in anhydrous and hydrated forms as well as the forms wherein the molecule is associated with inorganic or organic acids for the manufacture of a nutritional composition for the treatment or reduction of the incidence of muscle wasting in patients that suffer from cancer, HIV, COPD or surgical trauma.

2. Use according to claim 1, wherein the composition further comprises coenzyme Q10 (CoQ10) equivalents.

3. Use according to any one of claims claims 1-2, wherein the composition further comprises folate or equivalent thereof.

4. Use according to any one of claims 1-3 wherein the composition also comprises vitamin B6.

5. Use according to any one of claims 1-4 wherein the dose of betaine and/or equivalent thereof is between 50 and 50000 mg/day.

6. Use according to any one of claims 1-5 wherein the dose of CoQ10 and/or equivalent thereof is between 10-2000 mg/day.

7. A nutritional composition comprising betaine, CoQ10, at least 15 en% lipids, 10-70 en% digestible carbohydrates and 10-35 en% protein, wherein the protein comprises all essential amino acids and the source of protein is chosen from the group of; dairy proteins, egg proteins, plant proteins and hydrolysates thereof.

8. The nutritional composition according to claim 7 further comprising vitamin B6 and/or folate.

9. The nutritional composition according to claim 7 or 8 wherein the concentration of betaine and/or equivalent thereof is between 0.01 and 500 mg/ml and the concentration CoQ10 and/or equivalent thereof is between 0.002 and 20 mg/ml.

10. The nutritional composition according to any one of claims 7-9 wherein the concentration of folate is between 0.0005 and 0.05 mg/ml.

11. The nutritional composition according to any one of claims 7-10 wherein the concentration of vitamin B6 is between 0.001 and 0.07 mg/ml.

## Patentansprüche

1. Verwend ung von Betain, Dimethylglycin oder Betaineinschlusssalzen in wasserfreien und hydratisierten Formen sowie Formen, in denen das Molekül mit anorganischen oder organischen Säuren assoziiert ist, in der Herstellung einer Nahrungsmittelzusammensetzung für die Behandlung oder Verminderung des Auftretens von Muskelverfall bei Patienten, die an Krebs, HIV, COPD oder einem chirurgischen Trauma leiden.

2. Verwendung nach Anspruch 1, worin die Zusammensetzung des Weiteren Co-Enzym Q10- (CoQ10) Äquivalente umfasst.

3. Verwendung nach einem der Ansprüche 1 bis 2, worin die Zusammensetzung des Weiteren Folat oder Äquivalente davon umfasst.

4. Verwendung nach einem der Ansprüche 1 bis 3, worin die Zusammensetzung auch Vitamin B6 umfasst.

5. Verwendung nach einem der Ansprüche 1 bis 4, worin die Dosis von Betain und/oder Äquivalenten davon zwischen 50 und 50000 mg/Tag beträgt.

6. Verwendung nach einem der Ansprüche 1 bis 5, worin die Dosis von CoQ10 und/oder Äquivalenten davon zwischen 10-2000mg/Tag beträgt.

7. Nahrungsmittelzusammensetzung, die Betain, CoQ10, mindestens 15 en% Lipide, 10-70 en% verdauliche Kohlenhydrate und 10-35 en% Protein umfasst, worin das Protein alle essentiellen Aminosäuren umfasst und die Proteinquelle ausgewählt ist aus der Gruppe von Milchproteinen, Eierproteinen, Pflanzenproteinen und Hydrolysaten davon.

8. Nahrungsmittelzusammensetzung nach Anspruch 7, die des Weiteren Vitamin B6 und/oder Folat umfasst.

9. Nahrungsmittelzusammensetzung nach einem der Ansprüche 7 oder 8, worin die Konzentration an Betain und/oder Äquivalenten davon zwischen 0,01 und 500 mg/ml beträgt und die Konzentration an CoQ10 und/oder Äquivalenten davon zwischen 0,002 und 20 mg/ml beträgt.

10. Nahrungsmittelzusammensetzung nach einem der Ansprüche 7 bis 9, worin die Konzentration an Folat zwischen 0,0005 und 0,05 mg/ml beträgt.

11. Nahrungsmittelzusammensetzung nach einem der Ansprüche 7 bis 10, worin die Konzentration an Vitamin B6 zwischen 0,001 und 0,07 mg/ml beträgt.

## Revendications

1. Utilisation de bétaïne, de diméthylglycine ou de sels internes de bétaïne sous forme anhydre ou hydratée ainsi que sous les formes dans lesquelles la molécule est associée à des acides inorganiques ou organiques pour la fabrication d'une composition nutritionnelle destinée au traitement ou à la réduction d'une perte musculaire chez des patients souffrant d'un cancer, du VIH, d'une BPCO ou d'un traumatisme chirurgical.

2. Utilisation selon la revendication 1, dans laquelle la composition comprend en outre des équivalents de la coenzyme Q10 (CoQ10).

3. Utilisation selon l'une quelconque des revendications 1 à 2, dans laquelle la composition comprend un folate ou un équivalent de celui-ci.

4. Utilisation selon l'une quelconque des revendications 1 à 3, dans laquelle la composition comprend en outre de la vitamine B6.

5. Utilisation selon l'une quelconque des revendications 1 à 4, dans laquelle la dose de bétaïne et/ou de son équivalent se situe entre 50 mg/jour et 50 000 mg/jour.

6. Utilisation selon l'une quelconque des revendications 1 à 5, dans laquelle la dose de CoQ10 et/ou de son équivalent se situe entre 10 mg/jour et 2 000 mg/jour.

7. Composition nutritionnelle comprenant de la bétaïne, du CoQ10, au moins 15 en% de lipides, 10 à 70 en% de glucides digestibles et 10 à 35 en% de protéine, les protéines comprenant tous les acides aminés essentiels et la source de protéine étant choisie dans le groupe suivant : les protéines du lait, les protéines de l'oeuf, les protéines végétales et leurs hydrolysats.

8. Composition nutritionnelle selon la revendication 7, comprenant en outre de la vitamine B6 et/ou un folate.

9. Composition nutritionnelle selon la revendication 7 ou 8, dans laquelle la concentration de la bétaïne et/ou de son équivalent se situe entre 0,01 mg/ml et 500 mg/ml, et la concentration du CoQ10 et/ou de son équivalent se situe entre 0,002 mg/ml et 20 mg/ml.

10. Composition nutritionnelle selon l'une quelconque des revendications 7 à 9, dans laquelle la concentration du folate se situe entre 0,0005 mg/ml et 0,05 mg/ml.

11. Composition nutritionnelle selon l'une quelconque des revendications 7 à 10, dans laquelle la concentration de la vitamine B6 se situe entre 0,001 mg/ml et 0,07 mg/ml.
